# EUROPEAN PATENT SPECIFICATION

(11) **EP 2 131 791 B1**
(45) Date of publication and mention of the grant of the patent: **01.08.2012**
(21) Application number: 08744715.7
(22) Date of filing: 31.03.2008
(51) Int. Cl.: A61F 2/44, A61F 2/30, A61F 2/46

(54) **IMPLANT FACE PLATES**
GESICHTSIMPLANTATPLATTEN
PLATINES D'IMPLANT

(30) Priority: 03.04.2007 US 695883
(43) Date of publication of application: 16.12.2009
(73) Proprietor: Warsaw Orthopedic, Inc., Warsaw, IN 46581 (US)
(72) Inventor: WAUGH, Linsey Gardner, Memphis, Tennessee 38117 (US); EDIE, Jason A., Sandy, UT 84092 (US); SCHULTZ, Matthew D., Memphis, Tennessee 38135 (US)
(74) Representative: Viering, Jentschura & Partner
(86) International application number: PCT/US2008/058819
(87) International publication number: WO 2008/124355

(56) References cited:
- WO-A-2005/007041
- WO-A-2006/101837
- FR-A- 2 730 628
- US-A1- 2002 169 508
- US-B1- 6 629 998

## Description

### FIELD OF THE INVENTION

The present invention relates to an implantable medical device according to the preamble of claim 1. Generally, the present invention relates to the field of replacing portions of the human structural anatomy with medical implants.

### BACKGROUND

An implantable medical device of the initially-mentioned type is known, e.g., from US 6,629,998 B1.

Spinal discs between the endplates of adjacent vertebrae in a spinal column of the human body provide critical support. However, due to injury, degradation, disease or the like, these discs can rupture, degenerate and/or protrude to such a degree that the intervertebral space between adjacent vertebrae collapses as the disc loses at least a part of its support function. This can cause impingement of the nerve roots and severe pain. In some cases, surgical correction may be required.

Some surgical corrections include the removal of the natural spinal disc from between the adjacent vertebrae. In order to preserve the intervertebral disc space for proper spinal-column function, an implant member can be inserted between the adjacent vertebrae.

Some implant members employ anchor members that fix the implant member in place between the adjacent vertebrae. Over time, due to micro motion of the vertebrae relative to the implant member, the anchor members may loosen and start to back out of vertebrae.

What is needed is an implant that reduces or eliminates anchor member back-out. The implants disclosed herein address one or more deficiencies in the art.

### SUMMARY

To this end, the present invention provides an implantable medical device according to claim 1. Further embodiments of the implantable medical device are described in the dependent claims.

The implantable medical device is attachable to bone structure using an anchor member. The device includes an implant member including an anchor member aperture sized to receive the anchor member, and also includes a faceplate sized to overlap at least a portion of the anchor member aperture and inhibit back-out of the anchor member from the anchor member aperture. The faceplate is configured to snap onto the implantable member.

In an exemplary aspect, the present disclosure is directed to a faceplate for at least partially covering a plurality of anchor member apertures in an implantable member. The faceplate may include a main body sized to at least partially overlap the plurality of anchor member apertures of the implantable member and configured to inhibit anchor member back-out from the plurality of anchor member apertures.

Locking tabs are associated with the main body. E.g., the locking tabs are formed to at least partially elastically deform to engage the implantable member and secure the main body in place.

E.g., the implant member is introduced to a vertebral segment comprising an upper and a lower vertebra. Anchor members may be inserted through anchor member apertures in the implant member, the anchor members penetrating the upper and the lower vertebrae. The faceplate may be oriented to at least partially overlap the anchor member apertures, and the faceplate may be advanced until it snaps onto the implant member to inhibit anchor member back-out.

Further aspects, forms, embodiments, objects, features, benefits, and advantages of the present invention shall become apparent from the detailed drawings and descriptions provided herein.

### BRIEF DESCRIPTION OF THE DRAWINGS

Fig. 1 is an illustration of a lateral view of a segment of a lumbar spine.
Fig. 2 is an illustration of a lateral view of a spinal segment formed by two vertebrae with an exemplary implantable device disposed therebetween.
Fig. 3 is an illustration of a perspective view of the exemplary implant shown in Fig. 2.
Fig. 4 is an illustration of a partially exploded perspective view of the exemplary implant shown in Fig. 2.
Fig. 5 is an illustration of a perspective view of an exemplary face plate portion of the exemplary implant shown in Fig. 2.
Fig. 6 is an illustration of an exemplary insertion tool usable with the exemplary implantable device illustrated in Fig. 2.
Fig. 7 is an illustration of a perspective view of another exemplary implantable device.
Fig. 8 is an illustration of a perspective view of an exemplary face plate portion of the exemplary implant shown in Fig. 7
Fig. 9 is an illustration of a perspective view of another exemplary implantable device without the anchor members.
Fig. 10 is an illustration of a perspective view of an exemplary face plate portion of the exemplary implantable device in Fig. 9.
Fig. 11 is an illustration of a top view of the exemplary face plate portion shown in Fig. 10.
Fig. 12 is an illustration of a perspective view of another exemplary implantable device without the anchor members.
Fig. 13 is an illustration of an exploded perspective view of the exemplary implant shown in Fig. 12.
Fig. 14 is an illustration of a lateral view of the exploded exemplary implant shown in Fig. 9.

### DETAILED DESCRIPTION

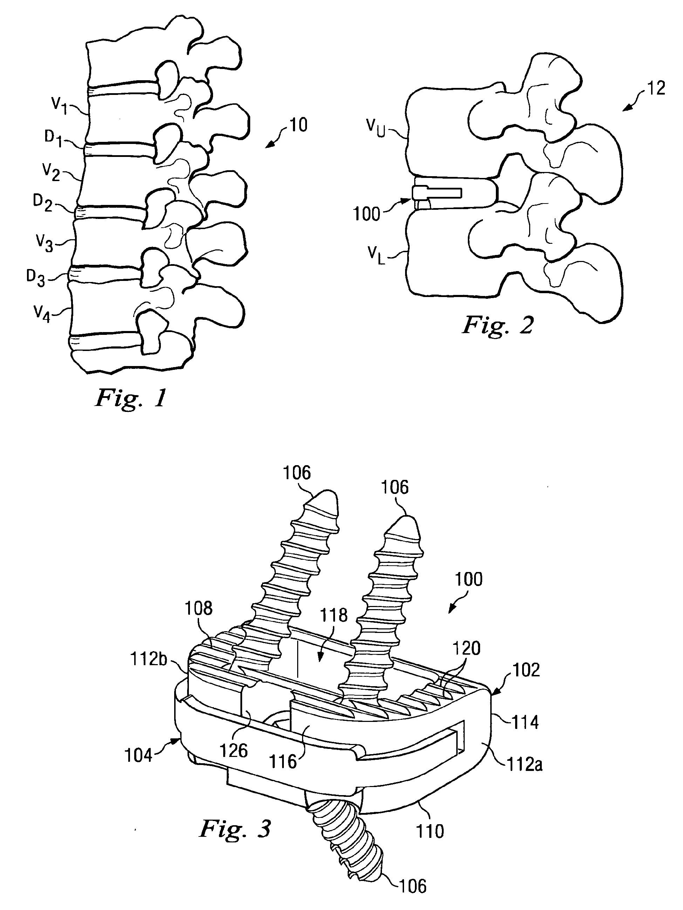

For the purposes of promoting an understanding of the principles of the invention, reference will now be made to the embodiments, or examples, illustrated in the drawings and specific language will be used to describe the same. Fig. 1 shows a lateral view of a portion of a spinal column 10, illustrating a group of adjacent upper and lower vertebrae V1, V2, V3, V4 separated by natural intervertebral discs D1, D2, D3. The illustration of four vertebrae is only intended as an example. Another example would be a sacrum and one vertebra.

For the sake of further example, two of the vertebrae will be discussed with reference to a spinal segment 12 shown in Fig. 2. An upper vertebra 14 and a lower vertebra 16, which may be any of the vertebrae V1, V2, V3, V4, define the spinal segment 12. Although the illustrations of Figs. 1 and 2 generally depict a lumbar vertebrae and a lumbar vertebral segment, it is understood that the devices, systems, and methods of this disclosure may also be applied to all regions of the vertebral column, including the cervical and thoracic regions.

Some types of disc arthroplasty require that some or the entire natural disc that would have been positioned between the two vertebrae 14, 16 be removed via a discectomy or a similar surgical procedure. Removal of the diseased or degenerated disc results in the formation of an intervertebral space between the upper and lower vertebrae 14, 16. Once the diseased or degenerated disc is removed, an implantable prosthetic device may be used to maintain the vertebral spacing and provide vertebral support. As shown in Fig. 2, an implantable device, referenced herein by the reference numeral 100, resides within the vertebral space. Sized to fit the disc space height in a manner similar to a natural intervertebral disc, such as any of discs D1-D4, the implantable device 100 provides support and stabilization to the vertebrae.

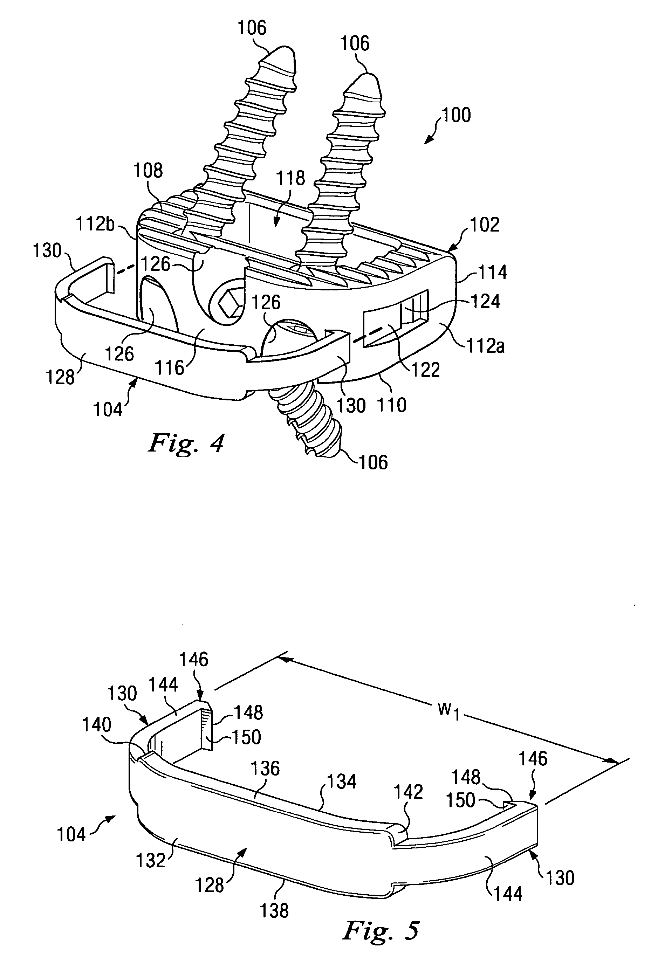

Figs. 3 and 4 show the implantable device 100 in greater detail. Fig. 3 shows a perspective view of the implantable device 100, while Fig. 4 shows a partially exploded view of the implantable device 100. Referring now to both Figs. 3 and 4, the implantable device includes an implant member 102, a face plate 104, and anchor members 106. The implant member 102 may include an upper surface 108, a lower surface 110, side surfaces, 112a-b, a rear surface 114 and a front surface 116. The upper and lower surfaces 108, 110 may be configured to interface with the load bearing endplates of the upper and lower vertebrae 14, 16, while the side, rear, and front surfaces 112a-b, 114, 116 extend between the upper and lower surfaces 108, 110. A hollow center 118 may allow placement of bone growth materials, such as allograft to promote bonding and fusion of the implantable device 100 to the adjacent vertebrae.

In the embodiment shown, the upper and lower surfaces 108, 110 include bone engaging features 120 configured to reduce slipping or movement of the implant member 102 relative to the vertebrae 14, 16. In the exemplary embodiment shown, the bone engaging features 120 are angled teeth that permit introduction into the disc space, but also restrict removal. These may be any other known bone engaging features, including protrusions and keels, among others. Alternatively, the upper and lower surfaces 108, 110 may be relatively smooth. The side surfaces 112a-b each include a recessed slot 122 (best seen in Fig. 4) configured to cooperate with an insertion tool (described below) that selectively connects to the implant member 102. Within the slot 122, attachment features, such as connecting impressions 124 (Fig. 4), are configured to provide a secure connection with the faceplate 104.

The front surface 116 and/or the upper and lower surfaces 108, 110 include anchor member apertures 126 that receive the anchor members 106 for attachment of the implant member 102 to the vertebral bodies 14, 16. In this exemplary embodiment, the anchor members 106 are bone anchor members. However, other anchor members are contemplated. The anchor members 106 extend through the anchor member apertures 126 in the front surface 116 and upper and lower surfaces 108, 110 and out the hollow center 118 and into the bearing endplates of the vertebrae 14, 16, thereby securely locating the implant member 102 entirely within the disc space. In this exemplary embodiment, the implant member 102 includes three apertures 126 - two angled to allow anchor members 106 to attach to an upper vertebral endplate and one angled to allow an anchor member 106 to attach to a lower vertebral endplate. However, in other embodiments, other numbers and configurations of anchor members 106 may be used.

To reduce the chance of the anchor members 106 backing out of the vertebral bodies over time and causing the implant member 102 to become loose, the face plate 104 is configured to extend across at least a portion of the anchor member apertures 126 and physically block anchor member back-out. Fig. 5, in addition to Figs. 3 and 4, shows the face plate 104 in detail. The exemplary face plate 104 shown in these figures includes a main body 128 and locking features that cooperate with the implant member 102 to secure the main body 128 in place over the anchor member apertures 126. In the embodiment shown, the locking features are compliant locking tabs 130 that protrude from the main body 128 and engage the implant member 102.

The main body 128 includes a front surface 132, an interfacing surface 134, respective top and bottom surfaces 136, 138, and first and second ends 140, 142. The interfacing surface 134 lies against the front surface 116 of the implant member 102 and inhibits the anchor members 106 from backing out by being physically located within the back-out path. In this exemplary embodiment, the main body 128 extends substantially the entire length of the front surface 116 in an arcing shape, from one side surface 112a to the other side surface 112b. Its height is less than the height of the front surface 116, and it overlaps only a portion of each of the anchor member apertures 126, as shown in Fig. 3. The locking tabs 130, in this exemplary embodiment, extend as cantilevers from the first and second ends 140, 142 of the main body 128, generally in the same direction forming a U-shape. In other embodiments, the locking tabs 130 extend from the top and bottom surfaces 136, 138 of the face plate 104. Each locking tab 130 includes a body portion 144 and a locking protrusion 146 extending from the body portion 144. The body portion 144 of each locking tab 130 is configured to fit within the corresponding recessed slots 122 formed in the side surfaces 112a-b of the implant member 102, and the locking protrusions 146 are sized and configured to interface with the corresponding connecting impressions 124 in the recessed slots 122. In the embodiment shown, the locking protrusions 146 are angled protrusions having a leading tapered surface 148 and a trailing locking surface 150.

In the embodiment shown, the faceplate 104 is configured to attach to the exterior sides 112a-b of the implant member 102. Thus, the faceplate 104 may be sized to span the width of the implant member 102, and may have an outer width W1. In other embodiments, the faceplate may be configured to attach to locations on the implant member 102 other than the exterior surfaces.

The faceplate 104 may be configured to snap onto the implant member 102, allowing simple installation once the anchor members 106 are placed during a surgical procedure. As used herein, the terms "snap onto," and "snaps onto" are intended to mean attachment using features of the different components without the use of separate fasteners, such as screws or staples. To do this, in the embodiment shown, the locking tabs 130 may be configured to elastically deform when placing the faceplate 104 on the implant member 102, and then snap into place, thereby securing the faceplate 104 onto the implant member 102 and physically limiting back-out of the anchor members 106 from the implant member 102. An example of this is provided further below.

When the implantable device 100 is a spacer as shown in Fig. 2, it is capable of being entirely contained within the disc space. Accordingly, anchor member locking features that reduce the chance of anchor member back-out, such as the locking tabs 130, also may be disposed entirely within the disc space. This reduces a chance of creating additional patient trauma that can occur if organs and tissue are able to easily contact the implantable device outside the disc space.

In the embodiment shown, the face plate 104 may be constructed in whole or in part of biocompatible materials of various types. Examples of face plate materials include, but are not limited to, non-reinforced polymers, carbon-reinforced polymer composites, PEEK and PEEK composites, shape-memory alloys, titanium, titanium alloys, cobalt chrome alloys, stainless steel, ceramics and combinations thereof. If the face plate 104 is made from radiolucent material, radiographic markers can be located on the face plate 104 to provide the ability to monitor and determine radiographically or fluoroscopically the location of the face plate in the spinal disc space. Resorbable materials also may be used. Examples of resorbable materials that may be used include, but are not limited to, polylactide, polyglycolide, tyrosine-derived polycarbonate, polyanhydride, polyorthoester, polyphosphazene, calcium phosphate, hydroxyapatite, bioactive glass, and combinations thereof.

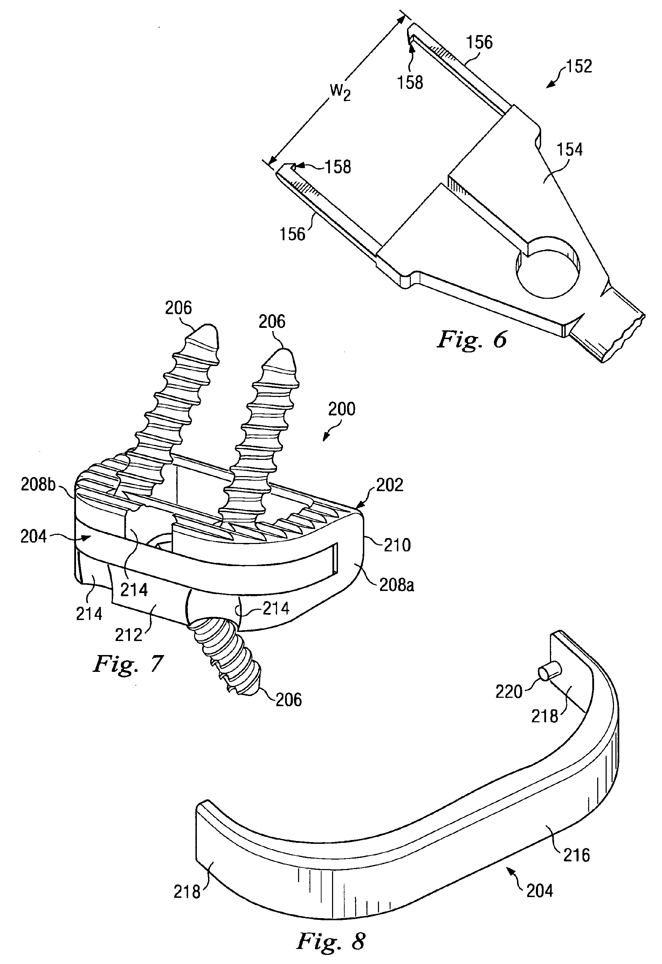

Fig. 6 shows an end of an insertion tool 152 for implanting the implant member 102. The insertion tool 152 includes a body portion 154 and two arms 156 configured to interface with the recessed slots 122 on the implant member side surfaces 112a-b. A locking protrusion 158 on each arm 156 is shaped and sized to cooperate with the connecting impressions 124 in the recessed slots 122. Accordingly, the insertion tool 152 and the faceplate 104 may be designed to cooperate with the same features on the implant member 102. The overall width W2 of the insertion tool 152 may be equal to or greater than the overall width W1 of the face plate 104. This may ensure that the faceplate 104 will fit properly within the disc space after the implant member 102 is introduced and placed in a patient with the insertion tool 152.

In alternative embodiments, instead of the locking protrusions 146, 158 being formed on the insertion tool 152 and/or on the faceplate 104, the locking protrusions are formed on the implant member 102 and the insertion tool 152 and/or faceplate 104 has connecting features, such as connecting impressions. Additionally, in some exemplary embodiments, the connecting impressions 124 are apertures, hooks or other protruding or recessed features. Further, in some exemplary embodiments, the insertion tool 152 and/or faceplate 104 connects to locations on the implant member 102 other than at the side surfaces 112a-b. For example, in some exemplary embodiments, the locking tabs 130 extend into bores formed in the surface of the implant member 102 adjacent the anchor member apertures 126. Other types and locations of attachments are contemplated.

In addition, although disclosed as being on the front surface 116 where the front surface is an anterior surface, it should be understood that the faceplate 104 may be disposed to cover any surface having an anchor member aperture 126 in the implantable device 102 to inhibit anchor member back-out. For example, some implant members 102 may be implanted posteriorly and may include anchor member apertures 126 on the rear side 114 or alternatively, on the lateral sides 112a-b. In these embodiments the face plate 104 may be disposed to cooperate with the apertures on those sides and inhibit anchor member back-out from those apertures.

Fig. 7 shows another exemplary embodiment of an implantable device, referenced herein by the numeral 200. The implantable device 200 may include an implant member 202, a faceplate 204, and anchor members 206. The implant member 202, the faceplate 204, and the anchor members 206 may include any or all of the features of the other embodiments described herein, and to reduce repetition, many of these features will not re-discussed with respect to the implantable device 200.

Like the implantable member 102, the implantable member 202 includes side surfaces 208a-b, a rear surface 210, and a front surface 212. Anchor member apertures 214 are shown formed in the front surface 212 and allow attachment to the bearing endplates of the vertebrae 14, 16 in Fig. 2. In this embodiment however, the side surfaces 208a-b and the front surface 212 include a recess formed therein (shown with faceplate 204 located therein) sized to receive the faceplate 204 so that the faceplate 204 sits flush with one of or all of the side surfaces 208a-b and the front surface 212. As with the other embodiments, the faceplate 204 overlaps the anchor member apertures 214 and inhibits anchor member back-out.

One exemplary embodiment of the faceplate 204 is shown in Fig. 8. In this exemplary embodiment, the faceplate 204 includes a main body 216 and locking tabs 218 that extend from the main body 216. In this embodiment, the main body height is substantially the same as the locking tab height. Locking protrusions 220 (only one is shown) extend inwardly from the locking tabs 218, generally facing each other. In this exemplary embodiment, the locking protrusions 220 are cylindrical projections configured to fit into cylindrical connecting impressions on the implant member 202. Again, as described above, the faceplate 204 is configured to snap onto the implant member 202 and inhibit anchor member back-out.

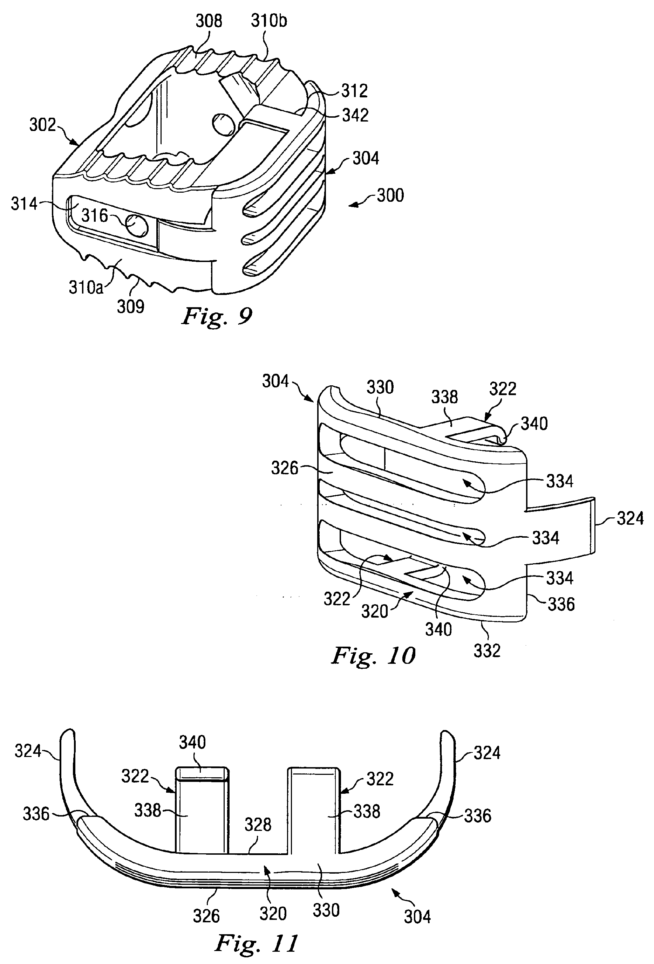

Fig. 9 shows another exemplary embodiment of an implantable device, referenced herein by the numeral 300. The implantable device 300 may include an implant member 302, a faceplate 304, and anchor members (not shown). The implant member 302, the faceplate 304, and the anchor members may include any or all of the features of the other embodiments described herein, and to reduce repetition, many of these features will not re-discussed with respect to the implantable device 300.

As can be seen in Fig. 9, the implant member 302 includes an upper surface 308, a lower surface 309, side surfaces, 310a-b, and a front surface 312. The side surfaces 310a-b each include a recessed slot 314 configured to cooperate with the above-described insertion tool 152 that selectively connects to the implant member 302. Within the slot 314, connecting impressions 316 are configured to provide a secure connection with the insertion tool. The front surface 312 includes anchor member apertures (not shown) that receive the anchor members for attachment of the implant member 302 to the vertebral bodies 14, 16.

The face plate 304 is shown in Figs. 10 and 11 and includes a main body 320, locking tabs 322, and securing tabs 324. The main body 320 includes a front surface 326, an interfacing surface 328, and respective top and bottom surfaces 330, 332. In the exemplary embodiment shown, the main body 320 includes viewing ports 334 that here are formed of slots extending across the main body 320. These viewing ports 334 allow an operating physician to view the previously inserted anchor members through the faceplate 304. The interfacing surface 328 lies against the front surface 312 of the implant member 302 and deters the anchor members from backing out by physically blocking them. The face plate 304 connects to the implant member 302 using, in this exemplary embodiment, the securing tabs 324 and locking tabs 322.

The securing tabs 324, in this exemplary embodiment, extend from sides 336 of the main body 320 and are configured to interface with and extend into the recessed slots 314 on the implant member 302. These securing tabs 324 act as guides to help orient and place the face plate 304 during assembly.

The locking tabs 322, in this exemplary embodiment, extend from the top and bottom surfaces 330, 332 of the main body 320 and are formed off-centerline, as best seen in Fig. 11. Each locking tab 322 includes a body portion 338 and a locking protrusion 340 extending from the body portion 338. The body portion 338 of each locking tab 322 is configured to fit within corresponding mating grooves 342 (best seen in Fig. 9) formed in the upper and lower surfaces 308, 309 of the implant member 302 and the locking protrusions 340 are sized and configured to interface with corresponding connecting impressions (not shown) in the mating grooves 342. The locking tabs 322, like those described with respect to other embodiments herein, may be configured to elastically deform when placing the face plate 304 on the implant member 302, and then snap into place, thereby securing the face plate 304 onto the implant member 302 and physically limiting back-out of the anchor members from the implant member 302.

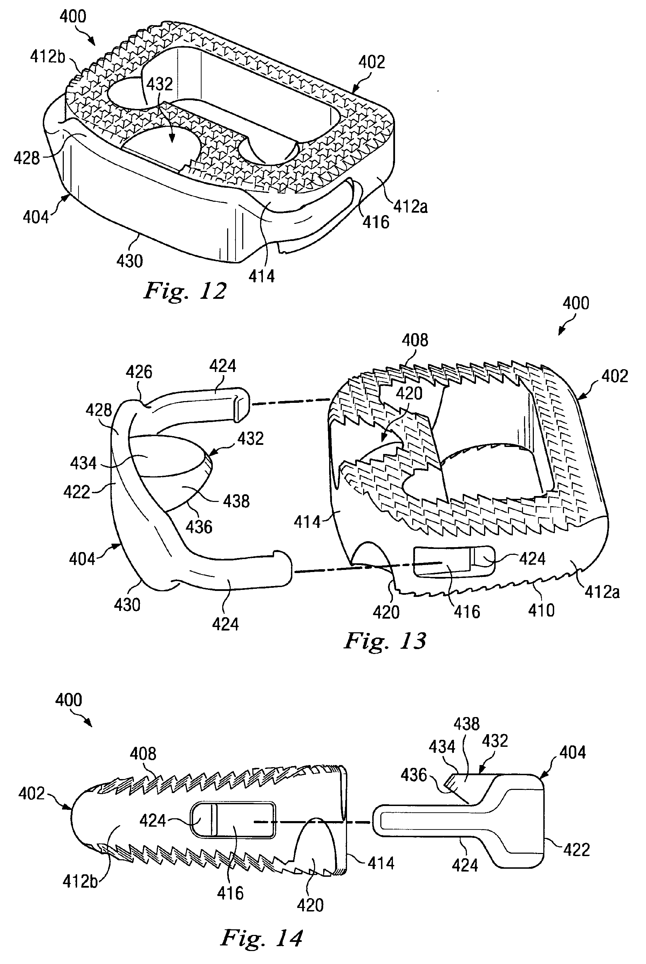

Yet another exemplary embodiment of an implantable device is shown in Figs. 12-14, referenced by the numeral 400. The implantable device 400 may include an implant member 402, a faceplate 404, and anchor members (not shown), each of which may include any or all of the features of the other embodiments described herein. To reduce repetition, many of these features will not be re-discussed with respect to the implantable device 400.

As can be seen in Figs. 12-14, the implant member includes an upper surface 408, a lower surface 410, side surfaces 412a-b, and a front surface 414. The side surfaces 412a-b each include a recessed slot 416 configured to cooperate with the above-described insertion tool 152 that selectively connects to the implant member 402. Within the slot 416, connecting impressions 424 are configured to provide a secure connection with the insertion tool and the faceplate 404. The front surface 414 and the upper and lower surfaces 408, 410 include anchor member apertures 420 that receive the anchor members for attachment of the implant member 402 to the vertebral bodies 14, 16.

The face plate 404 includes a main body 422 locking tabs 424. The main body 422 includes an interfacing surface 426 and respective top and bottom surfaces 428, 430. In the exemplary embodiment shown in Figs. 12-14, projecting protuberances 432 extend from the interfacing surface 426 of the main body 422 in the same general direction as the locking tabs 424. The projecting protuberances 432 are sized and shaped to at least partially penetrate into the anchor member apertures 420 and fit adjacent the anchor members. In some exemplary embodiments, the projecting protuberances 432 lie flat against or flush with the anchor members to substantially eliminate anchor member back-out. In these exemplary embodiments, the anchor members are held more tightly in place, and their movement is more limited than in the prior embodiments. In other exemplary embodiments, the projecting protuberances 432 do not lie flat against the anchor members, but still provide less room for back-out than the main body alone.

Referring now to Fig. 14, the projecting protuberances 432 include an outer surface 434 oriented to face away from the implant member 402, an inner surface 436 configured to face the anchor member, and a profile edge 438. Because the anchor member apertures 420 in the implant member 402 are angled, the inner surface 436 also is angled to lie flush with and adjacent to the end of the anchor member. The angle of the inner surface 436 is dependent on the implant member design and angle of the anchor members and anchor member apertures 420. In this exemplary embodiment, the anchor member apertures 420 extend through both the front surface 414 and the corresponding upper or lower surfaces 408, 410. Therefore, in the embodiment shown, the outer surface 434 is oriented outside the anchor member aperture 420 and lies flush with the corresponding upper or lower surface 408, 410. In other embodiments, where the anchor member aperture 420 is disposed only through the side surface 412, the front surface 414, or the rear surface 415, the projecting protuberances 432 extend into and may be fully encompassed by the anchor member aperture 420.

For ease of explanation, the faceplate 404 is shown with only a single projecting protuberance 432. However, in other embodiments, the main body 422 includes a projecting protuberance 432 that corresponds with each anchor member aperture 420 in the implant member 402. Accordingly, the main body 422 with the projecting protuberances 432 deters the anchor members from backing out by physically blocking them. As in other embodiments shown herein, the face plate 404 connects to the implant member 402 using, in this exemplary embodiment, the locking tabs 424.

The locking tabs 424, like those described with respect to other embodiments herein, may be configured to elastically deform when placing the face plate 404 on the implant member 402, and then snap into place, thereby securing the face plate 404 onto the implant member 402 and physically limiting back-out of the anchor members from the implant member 402.

A method of implantation is described below with reference to the first embodiment of the implantable device 100 disclosed herein. However, the method of implantation is intended to be equally applicable to all the disclosed embodiments.

The spacers may be implanted between the vertebrae 14, 16 using any common approach, including an anterior approach, a posterior approach, a posterior transforaminal approach, a far lateral approach, a direct lateral approach, among others. According to at least one of these approaches, an incision, such as a midline incision, may be made in the patient's back and some or all of the affected disc and surrounding tissue may be removed via the foramina. The endplate surface of the vertebra 14 may be milled, rasped, or otherwise resected to match the profile of the upper surface 108 of the implant member 102, to normalize stress distributions on the endplate surface of the vertebra 14, and/or to provide initial fixation prior to bone ingrowth. The preparation of the endplate of vertebra 14 may result in a flattened surface or in surface contours such as pockets, grooves, or other contours that may match the bone engaging features 120 on the upper surface 108. The endplate of the vertebra 16 may be similarly prepared to engage the lower surface 110 of the implant member 102 to the extent allowed by the exiting nerve root and the dorsal root ganglia. In some procedures, the natural facet joints of vertebrae 14, 16 may be trimmed or removed to provide access to the disc space.

The implant member 102 then may be attached to the insertion tool 152 and introduced into the intervertebral disc space between the upper and lower vertebrae 14, 16. Once properly positioned, the insertion tool 152 may be removed from the implant member 102 and anchor members 106 may be driven through the anchor member apertures 126 into the vertebral endplates. Alternatively, the anchor members 106 may be driven into the anchor member apertures 126 before the insertion tool 152 is removed. Once the anchor members 106 are driven into the bone, the anchor member apertures 126 may be covered with the faceplate 104 to prevent anchor member back-out.

The faceplate 104 may be introduced to the implant member 102 so that the locking tabs 130 align with the recessed slots 122. While advancing the faceplate 104, the tapered surfaces 148 of the locking protrusions 146 contact the implant member 102 and are elastically forced apart. Further advancement slides the locking protrusions 146 along the recessed slot 122 until the locking protrusions 146 reach the connecting impressions 124. Once there, the faceplate 104 snaps onto the implant member 102 because the locking tabs 130 deform toward their original positions as the locking protrusions 146 at least partially snap into the connecting impressions 124. The locking surface 150 on the locking protrusion 146 engages against the connecting impressions 124 and resists detaching the faceplate 104 from the implant member 102. The main body 128 of the faceplate 102 is now oriented to overlap at least a part of the anchor member apertures 126, and the surgical process may be completed in a manner known in the art.

Over time, due to micromotion of the vertebrae and the implant member 102, the anchor members 106 may loosen slightly. However, the anchor members 106 may be unable to back-out because they come into contact with the main body 128 of the faceplate 104. Thus, anchor member back-out is inhibited by the faceplate 104. In some embodiments, after the faceplate 104 snaps onto the implant member 102, additional locking features, such as a screw fastener, may be used to further attach the faceplate 104 to the implant member 102.

In some embodiments, the implant member or individual components of the implantable member are constructed of solid sections of bone, other tissues, or resorbable materials. Tissue materials include, but are not limited to, synthetic or natural autograft, allograft or xenograft, and may be resorbable or non-resorbable in nature. Examples of other tissue materials include, but are not limited to, hard tissues, connective tissues, demineralized bone matrix and combinations thereof. Examples of resorbable materials that may be used include, but are not limited to, polylactide, polyglycolide, tyrosine-derived polycarbonate, polyanhydride, polyorthoester, polyphosphazene, calcium phosphate, hydroxyapatite, bioactive glass, and combinations thereof. The implantable device may be solid, porous, spongy, perforated, drilled, and/or open.

In some circumstances, it is advantageous to pack all or a portion of the hollow center of the implant member with a suitable osteogenetic material or therapeutic composition. Osteogenic materials include, without limitation, autograft, allograft, xenograft, demineralized bone, synthetic and natural bone graft substitutes, such as bioceramics and polymers, and osteoinductive factors. A separate carrier to hold materials within the device can also be used. These carriers can include collagen-based carriers, bioceramic materials, such as BIOGLASS®, hydroxyapatite and calcium phosphate compositions. The carrier material may be provided in the form of a sponge, a block, folded sheet, putty, paste, graft material or other suitable form. The osteogenetic compositions may include an effective amount of a bone morphogenetic protein, transforming growth factor β1, insulin-like growth factor 1, platelet-derived growth factor, fibroblast growth factor, LIM mineralization protein (LMP), and combinations thereof or other therapeutic or infection resistant agents, separately or held within a suitable carrier material.

## Claims

1. An implantable medical device (100, 200, 300, 400) attachable to bone structure using an anchor member (106, 206), comprising
an implant member (102, 202, 302, 402) to be implanted between an upper vertebrae (V_{U}) and a lower vertebrae (V_{L}), having an upper surface (108, 308, 408), a lower surface (110, 309, 410), side surfaces (112a-b, 208a-b, 310a-b, 412a-b), a rear surface (114, 210) and a front surface (116, 212, 312, 414), the upper and lower surfaces (108, 308, 408, 110, 309, 410) being configured to interface with an endplate of the upper vertebrae (V_{U}) and an endplate of the lower vertebrae (V_{L}), respectively, while the side, rear and front surfaces (112a-b, 208a-b, 310a-b, 412a-b, 114, 210, 116, 212, 312, 414) extend between the upper and lower surfaces (108, 308, 408, 110, 309, 410), the implant member (102, 202, 302, 402) including an anchor member aperture (126, 214, 420) on one of the side surfaces (112a-b, 208a-b, 310a-b, 412a-b), the rear surface (114, 210) and the front surface (116, 212, 312, 414), which is sized to receive the anchor member (106, 206);
**characterized by**
a faceplate (104, 204, 304, 404) having a main body (128, 216, 320, 422) sized to overlap at least a portion of the anchor member aperture (126, 214, 420) so as to inhibit back-out of the anchor member (106, 206) from the anchor member aperture (126, 214, 420), the faceplate (104, 204, 304, 404) further having two compliant locking tabs (130, 218, 322, 424) extending from first and second ends (140, 142) or top and bottom surfaces (330, 332) of the main body (128, 216, 320, 422) and being configured to snap onto the implantable member (100, 200, 300, 400) so as to extend along opposing surfaces of the implantable member (102, 202) which are adjacent to the surface having the anchor member aperture (126, 214, 420).

2. The device (100, 200, 300, 400) of claim 1, wherein the implant member (102, 202, 302, 402) comprises faceplate attachment features onto which the compliant locking tabs (130, 218, 322, 424) snap.

3. The device (100, 200, 300, 400) of claim 2, wherein the faceplate attachment features include connecting impressions (124, 316, 424) in the implant member (102, 202, 302, 402).

4. The device (100, 200, 300, 400) of claim 3, wherein the implant member (102, 202, 302, 402) includes recesses (122, 314, 416) formed therein and the connecting impressions (124, 316, 424) are formed in the recesses (122, 314, 416).

5. The device (100, 200, 300, 400) of claim 3, wherein the faceplate attachment features are configured to cooperate with an insertion tool (152) for implantation of the implant member (102, 202, 302, 402).

6. The device of claim 1, wherein the compliant locking tabs (130, 218, 322, 424) extend from the main body (128, 216, 422) so that the main body (128, 216, 422) and the locking tabs (130, 218, 424) form a U-shape.

7. The device (200) of claim 1, wherein the implant member (202) includes a recessed portion, and wherein the main body (216) fits within the recessed portion.

8. The device (400) of claim 1, wherein the faceplate (404) further comprises projecting protuberances (432) extending from the main body (422) toward the implant member (402), the projecting protuberances (432) extending at least partially into the anchor member apertures (420).

9. The device (400) of claim 8, wherein the projecting protuberances (432) have an inner surface (436) configured to lie adjacent the anchor member.

10. The device (400) of claim 9, wherein the inner surface (436) is angled relative to the main body (422).

11. The device (300) of claim 1, wherein the faceplate (304) further comprises securing tabs (324) extending from the main body (320), the securing tabs (324) being configured to guide and orient the faceplate (304) onto the implant member (302).

12. The device (300) of claim 11, wherein the main body (304) comprises viewing ports (334) extending through the main body (304) and located to allow visual viewing of the anchor member aperture.

## Patentansprüche

1. Eine implantierbare medizinische Vorrichtung (100, 200, 300, 400), welche an einer Knochenstruktur anbringbar ist unter Verwendung eines Anker- bzw. Befestigungselements (106, 206), aufweisend
ein Implantat-Element (102, 202, 302, 402), das zwischen einen oberen Wirbel (V_{U}) und einen unteren Wirbel (V_{L}) zu implantieren ist, mit einer oberen Fläche (108, 308, 408), einer unteren Fläche (110, 309, 410), Seitenflächen (112a-b, 208a-b, 310a-b, 412a-b), einer hinteren Fläche (114, 210) und einer vorderen Fläche (116, 212, 312, 414), wobei die obere Fläche und die untere Fläche (108, 308, 408, 110, 309, 410) konfiguriert sind, um mit/bzgl. einer Endplatte des oberen Wirbels (V_{U}) bzw. einer Endplatte des unteren Wirbels (V_{L}) eine Grenz- oder Berührungsfläche zu bilden bzw. sich an diese anzuschließen, während die seitliche, hintere und vordere Fläche (112a-b, 208a-b, 310a-b, 412a-b, 114, 210, 116, 212, 312, 414) sich zwischen der oberen Fläche und der unteren Fläche (108, 308, 408, 110, 309, 410) erstrecken, wobei das Implantat-Element (102, 202, 302, 402) eine Befestigungselement-Öffnung (126, 214, 420) an einer von den Seitenflächen (112a-b, 208a-b, 310a-b, 412a-b), der hinteren Fläche (114, 210) und der vorderen Fläche (116, 212, 312, 414) aufweist, welche dimensioniert bzw. bemessen ist, um das Befestigungselement (106, 206) aufzunehmen;
**gekennzeichnet durch**
eine Abdeckung (104, 204, 304, 404) mit einem Hauptkörper (128, 216, 320, 422), welcher bemessen bzw. dimensioniert ist, um zumindest einen Abschnitt der Befestigungselement-Öffnung (126, 214, 420) zu überlappen, um ein Zurücktreten des Befestigungselements (106, 206) aus der Befestigungselement-Öffnung (126, 214, 420) zu verhindern, wobei die Abdeckung (104, 204, 304, 404) ferner zwei nachgiebige Arretierlaschen bzw. Lappen (130, 218, 322, 424) aufweist, welche sich von einem ersten Ende bzw. einem zweiten Ende (140, 142) oder von einer oberen Fläche bzw. einer unteren Fläche (330, 332) des Hauptkörpers (128, 216, 320, 422) aus erstrecken und konfiguriert sind, um auf das implantierbare Element (100, 200, 300, 400) aufzuschnappen bzw. aufzurasten, um sich entlang gegenüberliegender Flächen des implantierbaren Elements (102, 202) zu erstrecken, welche benachbart zu der Fläche sind, welche die Befestigungselement-Öffnung (126, 214, 420) hat.

2. Die Vorrichtung (100, 200, 300, 400) nach Anspruch 1, wobei das Implantat-Element (102, 202, 302, 402) Abdeckungs-Befestigungsmerkmale aufweist, auf welche die nachgiebigen Arretierlaschen (130, 218, 322, 424) schnappen bzw. rasten.

3. Die Vorrichtung (100, 200, 300, 400) nach Anspruch 2, wobei die Abdeckungs-Befestigungsmerkmale Verbindungs-Vertiefungen (124, 316, 424) in dem Implantat-Element (102, 202, 302, 402) aufweisen.

4. Die Vorrichtung (100, 200, 300, 400) nach Anspruch 3, wobei das Implantat-Element (102, 202, 302, 402) Aussparungen (122, 314, 416) aufweist, welche darin geformt sind, wobei die Verbindungs-Vertiefungen (124, 316, 424) in den Aussparungen (122, 314, 416) geformt sind.

5. Die Vorrichtung (100, 200, 300, 400) nach Anspruch 3, wobei die Abdeckungs-Befestigungsmerkmale konfiguriert sind, um mit einem Einsetzwerkzeug (152) zum Implantieren des Implantat-Elements (102, 202, 302, 402) zu kooperieren bzw. zusammenzuwirken.

6. Die Vorrichtung nach Anspruch 1, wobei die nachgiebigen Arretierlaschen (130, 218, 322, 424) sich von dem Hauptkörper (128, 216, 422) aus erstrecken, sodass der Hauptkörper (128, 216, 422) und die Arretierlaschen (130, 218, 424) eine U-Form bilden.

7. Die Vorrichtung (200) nach Anspruch 1, wobei das Implantat-Element (202) einen ausgesparten Abschnitt aufweist, und wobei der Hauptkörper (216) in den ausgesparten Abschnitt passt.

8. Die Vorrichtung (400) nach Anspruch 1, wobei die Abdeckung (404) ferner vorspringende Vorsprünge (432) hat, welche sich von dem Hauptkörper (422) in Richtung zu dem Implantat-Element (402) erstrecken, wobei die vorspringenden Vorsprünge (432) sich zumindest teilweise in die Befestigungsmittel-Öffnungen (420) erstrecken.

9. Die Vorrichtung (400) nach Anspruch 8, wobei die vorspringenden Vorsprünge (432) eine innere Fläche (436) haben, welche konfiguriert ist, um benachbart zu dem Befestigungselement zu liegen.

10. Die Vorrichtung (400) nach Anspruch 9, wobei die innere Fläche (436) bzgl. des Hauptkörpers (422) schräg bzw. in einem Winkel steht.

11. Die Vorrichtung (300) nach Anspruch 1, wobei die Abdeckung (304) ferner Sicherungslaschen (324) aufweist, welche sich von dem Hauptkörper (320) aus erstrecken, wobei die Sicherungslaschen (324) konfiguriert sind, um die Abdeckung (304) auf das Implantat-Element (302) zu führen und auszurichten.

12. Die Vorrichtung (300) nach Anspruch 11, wobei der Hauptkörper (304) Beobachtungsoffnungen (334) hat, welche sich durch den Hauptkörper (304) hindurch erstrecken und angeordnet sind, um ein visuelles Beobachten bzw. Betrachten der Befestigungselement-Öffnung zu ermöglichen.

## Revendications

1. Dispositif médical implantable (100, 200, 300, 400) pouvant être fixé à une structure osseuse à l'aide d'un élément formant ancrage (106, 206), comprenant :
un élément formant implant (102, 202, 302, 402) à implanter entre une vertèbre supérieure (V_{U}) et une vertèbre inférieure (V_{L}), ayant une surface supérieure (108, 308, 408), une surface inférieure (110, 309, 410), des surfaces latérales (112a-b, 208a-b, 310a-b, 412a-b), une surface arrière (114, 210) et une surface avant (116, 212, 312, 414), les surfaces supérieure et inférieure (108, 308, 408, 110, 309, 410) étant conçues pour s'interfacer avec une plaque d'extrémité de la vertèbre supérieure (V_{U}) et une plaque d'extrémité de la vertèbre inférieure (V_{L}) respectivement, alors que les surfaces latérales arrière et avant (112a-b, 208a-b, 310a-b, 412a-b, 114, 210, 116, 212, 312, 414) s'étendent entre les surfaces supérieure et inférieure (108, 308, 408, 110, 309, 410), l'élément formant implant (102, 202, 302, 402) comprenant une ouverture d'élément formant ancrage (126, 214, 420) sur l'une parmi les surfaces latérales (112a-b, 208a-b, 310a-b, 412a-b), la surface arrière (114, 210) et la surface avant (116, 212, 312, 414), qui est dimensionnée pour recevoir l'élément formant ancrage (106, 206) ;
**caractérisé par** :
une plaque frontale (104, 204, 304, 404) ayant un corps principal (128, 216, 320, 422), dimensionnée pour recouvrir au moins une partie de l'ouverture d'élément formant ancrage (126, 214, 420) afin d'empêcher le retrait de l'élément formant ancrage (106, 206) de l'ouverture d'élément formant ancrage (126, 214, 420), la plaque frontale (104, 204, 304, 404) ayant en outre deux languettes de blocage souples (130, 218, 322, 424) s'étendant à partir des première et seconde extrémités (140, 142) ou bien des surfaces supérieure et inférieure (330, 332) du corps principal (128, 216, 320, 422) et étant conçues pour s'encliqueter sur l'élément implantable (100, 200, 300, 400) afin de s'étendre le long des surfaces opposées de l'élément implantable (102, 202) qui sont adjacentes par rapport à la surface ayant l'ouverture d'élément formant ancrage (126, 214, 420).

2. Dispositif (100, 200, 300, 400) selon la revendication 1, dans lequel l'élément formant implant (102, 202, 302, 402) comprend des caractéristiques de fixation de plaque frontale sur lesquelles les languettes de blocage souples (130, 218, 322, 424) s'encliquettent.

3. Dispositif (100, 200, 300, 400) selon la revendication 2, dans lequel les caractéristiques de fixation de plaque frontale comprennent des impressions de raccordement (124, 316, 424) dans l'élément formant implant (102, 202, 302, 402).

4. Dispositif (100, 200, 300, 400) selon la revendication 3, dans lequel l'élément formant implant (102, 202, 302, 402) comprend des évidements (122, 314, 416) formés à l'intérieur de ce dernier et les impressions de raccordement (124, 316, 424) sont formées dans les évidements (122, 314, 416).

5. Dispositif (100, 200, 300, 400) selon la revendication 3, dans lequel les caractéristiques de fixation de plaque frontale sont conçues pour coopérer avec un outil d'insertion (152) pour l'implantation de l'élément formant implant (102, 202, 302, 402).

6. Dispositif selon la revendication 1, dans lequel les languettes de blocage souples (130, 218, 322, 424) s'étendent à partir du corps principal (128, 216, 422) de sorte que le corps principal (128, 216, 422) et les languettes de blocage (130, 218, 424) forment un U.

7. Dispositif (200) selon la revendication 1, dans lequel l'élément formant implant (202) comprend une partie évidée, et dans lequel le corps principal (216) s'adapte à l'intérieur de la partie évidée.

8. Dispositif (400) selon la revendication 1, dans lequel la plaque frontale (404) comprend en outre des protubérances en saillie (432) s'étendant à partir du corps principal (422) vers l'élément formant implant (402), les protubérances en saillie (432) s'étendant au moins partiellement dans les ouvertures d'élément formant ancrage (420).

9. Dispositif (400) selon la revendication 8, dans lequel les protubérances en saillie (432) ont une surface interne (436) conçue pour être adjacente à l'élément formant ancrage.

10. Dispositif (400) selon la revendication 9, dans lequel la surface interne (436) est coudée par rapport au corps principal (422).

11. Dispositif (300) selon la revendication 1, dans lequel la plaque frontale (304) comprend en outre des languettes de fixation (324) s'étendant à partir du corps principal (320), les languettes de fixation (324) étant conçues pour guider et orienter la plaque frontale (304) sur l'élément formant implant (302).

12. Dispositif (300) selon la revendication 11, dans lequel le corps principal (304) comprend des orifices de visualisation (334) s'étendant à travers le corps principal (304) et positionnés pour permettre l'observation visuelle de l'ouverture d'élément formant ancrage.
